# EUROPEAN PATENT SPECIFICATION

(11) **EP 0 565 585 B1**
(45) Date of publication and mention of the grant of the patent: **10.04.1996**
(21) Application number: 92902438.8
(22) Date of filing: 30.12.1991
(51) Int. Cl.: A61M 1/00, A61M 1/14

(54) **HEMODIALYSIS APPARATUS**
HÄMODIALYSEAPPARAT
APPAREIL D'HEMODIALYSE

(30) Priority: 03.01.1991 DK 6/91
(43) Date of publication of application: 20.10.1993
(73) Proprietor: PEDERSEN, Robert, Smith, DK-6720 Fano (DK); AUNSHOLT, Niels, Aage, DK-8210 Hasle (DK); LYKKE, Borge, Nielsen, DK-8270 Hojbjerg (DK)
(72) Inventor: PEDERSEN, Robert, Smith, DK-6720 Fano (DK); AUNSHOLT, Niels, Aage, DK-8210 Hasle (DK); LYKKE, Borge, Nielsen, DK-8270 Hojbjerg (DK)
(74) Representative: Pedersen, Soeren Skovgaard
(86) International application number: DK9100407
(87) International publication number: WO9211878

(56) References cited:
- EP-A- 0 122 604
- EP-A- 0 343 267
- DD-A- 112 601
- DE-A- 3 019 353
- DE-A- 3 909 967
- GB-A- 2 106 993
- SE-B- 452 021
- US-A- 4 781 548

## Description

The present invention relates to a hemodialyzer apparatus comprising an inlet for blood, an outlet for treated blood, a blood flow path with an adjustable blood pump, a pressure gauge for monitoring the blood pressure, an air detector for detecting air in the blood flow path, an artificial kidney or filtration device, such as a filter column comprising at least one filter for passage of liquid, plasma or other substance from the blood, a dialysate channel connected to the dialysate outlet of the artificial kidney or filtration device, at least one adjustable dialysis pump and a receptacle for the dialysate.

A hemodialyzer apparatus of this art is known e.g. from EP A1-0 122 604 and is used in hospitals for cleaning of and nourishment supply to the patient's blood.

However, it presents a problem that the known apparatus are rather space consuming, and to have a reasonable utilization the pumps are allowed to work with so high output per time unit, that a treatment of a patient, whose blood should be cleansed e.g. due to insufficient function of the kidneys is executed within the range of 5 hours for an adult patient. The patient shall often have such treatment repeated every other or every third day. Furthermore in connection with the treatments dialysate supply is used, which is based on water; commonly water from the local water supply which initially has undergone a water treatment and afterwards added different suitable electrolytes and/or physiological substances or mixtures, which should be added to the patient's blood. Such water treatment plants are often large and expensive.

However, it is a purpose to provide a hemodialyzer apparatus, which is distinguished by to be inexpensive and further transportable in a convenient manner, such that it can be used at a patient's bed and relatively easily can be transported together with this and the patient to different sections in a hospital.

It turns out that said task in a surprisingly simple manner can be fulfilled according to the invention in that the blood pump (S) is a stepless variable pump and that the dialysis pump is a stepwise adjustable pump (S). This solution implies a large number of advantages, individually as well as in combination.

By means of the apparatus according to the invention it has thus become much easier to put patients having an extremely or critically low pressure into dialysis treatment which occurs in certain cases in intensive care wards, and where it is also desirable to let the dialysis treatment continue round the clock. For this purpose the pump outlet shall of course be approximately five times lower per time unit than by the hitherto known dialyzer apparatus. For round-the-clock treatment of patients having extremely low blood pressure the pump outlet must be moderate and still exactly adjustable.

By an apparatus for a 24-hour treatment or more the blood pump should have an outlet up to approximately 20 l per hour, i.e. adjustable almost from zero to approximately 20 l per hour. Said adjustment is known and could be achieved by known stepless variable blood pumps, which could be of different kinds, i.e. tube pumps as well as e.g. diaphragm pumps.

The outlet of the dialysis pump or pumps should, however, be considerably less, namely less than a tenth of the outlet of the blood pump, and to achieve an accurate withdrawal of plasma, liquid or another substance from the blood through the artificial kidney or a filtration device, the adjustability must be adequate. This, surprisingly enough, can be achieved by a stepwise adjustment of the pump outlet. It must be taken into consideration, that the blood pressure in certain situations can be extremely low, as low as approximately 2660,4 Pa (20 mm Hg) must be foreseen, it is easily recognized that the stepwise adjustment should be possible down to steps in the range of 1 ml per hour and with outlet of the dialysis pump from 0 to 2000 ml per hour. According to experience it can be necessary to arrange the pumps with a tachometer, which generates a return signal to the pump controlling devices and for that purpose is arranged on a shaft of the dialysis pump.

A hemodialyzer apparatus might also be used in the treatment of children. In such cases it is an advantage that the pump be arranged entirely or partly interchangeable or changeable between e.g. two pump outlets.

It should be noted, that from patients in e.g. intensive care it is usual to take dialysate samples during dialysis treatment, i.e. samples taken are examined, such that the quantity of waste substances are known. The result is used for comparative reasons in the dialyzer apparatus, if it is equipped with a microprocessor or computer for controlling and monitoring. Adjustments of the dialyzer apparatus are made in accordance with the results of said dialysis samples.

It appears that the apparatus according to the invention can be designed with small dimensions, and further that ready made dialysate preparations in plastic bags can be used with advantage, whereby the desired transportability of the dialyzer apparatus is achievable and not requiring a hospital having a water treatment plant. The apparatus can also be used at the site of a catastrophe for first aid treatment of patients having kidney failure.

According to the invention it is advantageous to provide designs which are characterized in that there are two dialysis pumps in the dialysis channel, arranged step-wise variable, the pump speed of the first pump is adjusted to present a predetermined draw volume per time unit from the outlet of the artificial kidney or filtration device, and the other dialysis pump is adjusted to supply a predetermined volume per time unit of fresh or cleansed dialysate to the dialysis inlet of the artificial kidney or filtration device.

Especially here is the invention advantageous when e.g. plastic bags are used for receiving of the dialysate from the apparatus and with ready made dialysate preparations ready to be supplied to the apparatus, and the apparatus is characterized in that the dialysate from the first dialysis pump is conveyed to a receptacle, such as a receptacle made of plastic sheeting, connected with the weighing mechanism for weighing of the dialysate quantity, and that fresh or cleansed dialysate from a storage receptacle, such as a storage bag of plastic sheeting and filled with the dialysate, also connected with the weighing mechanism, via the other dialysis pump the output of which is adjusted determined by the two changing quantities - one reducing the other raising - being kept equal per time unit or increasing in a pre-set manner, resulting in withdrawal fluid from the patient via the filtration device.

It appears that the invention is easily modified to further applications of different, but obvious art, and an easy and fast switch is possible. According to the invention such embodiments are partly characterized in that the artificial kidney or filtration device is provided with filter for plasmaforese and/or plasma replacement, and that the other dialysis pump is adjusted synchronously with the first dialysis pump, and partly that the artificial kidney or filtration device has one for the purpose suitable, such as plasmaforese filtration providing, filter member, and that the first dialysis pump supplies substitution fluid to the patient. Alternatively dialysate can be led to an adsorption providing, such as immuno adsorption providing, such as antibodies and cholesterol removing, filter member, from the outlet of the filter member the treated, possible further treated, dialysate is conveyed to the arterial inlet of the artificial kidney or filtration device, and partly characterized in that the other dialysis pump is in the dialysate flow path between the dialysate outlet of the treated, possible further treated dialysate and the dialysate inlet of the artificial kidney or filtration device, and that the two dialysis pumps are adjusted to work synchronously, and further characterized in that artificial kidney or filtration device is arranged to eliminate liquid, preferably water drawing, from the passing blood, and that the dialysate via the first dialysis pump, which is adjusted to pass a determined liquid volume per time unit, is conveyed to a receptacle, such as a receptacle connected with a weighing mechanism for determination of received liquid volume, preferably a receptacle of plastic sheeting.

According to the invention the hemodialyzer apparatus can be safeguarded against important malfunctioning states, such as the presence of too much air in the blood path, by a shut off valve being present in the blood flow path, such as a tube clamp, which controllingly is in connection with the air detector and is closed by this, when presence of an air volume is detected, above a predetermined maximum, and further against run out or failing fresh dialysate preparation thereby that weighing mechanism for the receptacle with preparation is arranged with an alarm device, giving alarm for an outlet quantity, which is below a predetermined quantity, which should be conveyed to the dialysate flow path per time unit.

The following advantages are achieved or can be achieved with dialyzer apparatus according to the invention:
1) Continuous hemodialysis treatment, including hemofiltration if necessary, which day and night removes residue products and liquid.
2) Accurately adapted withdrawal of liquid and plasma.
3) Possibility to dose medicines accurately.
4) Independency of water treatment or purifying plants.
5) Independency of connection to the public power supply, as battery operation is possible due to low power consumption.
6) Easy to transport.
7) Application of ready made dialysate preparation.
8) Negligible chances of infection, as the blood path as well as the dialysate path are closed circulations.
9) Easy to switch between adult and children.
10) Negligible need for sterilization.
11) Adapted to electronic controlling.
12) Easy to switch between different kinds of treatments, and the apparatus is less expensive than the apparatus known hitherto.

The present invention will now be described in detail with reference to the accompanying drawings, in which:

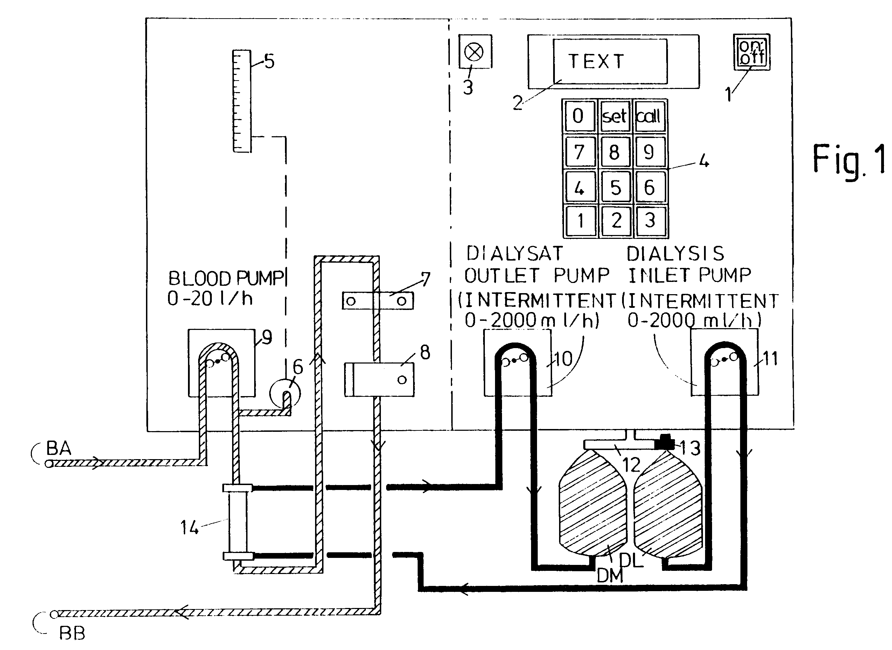

Fig. 1 is a schematical view of an embodiment of the hemodialyzer apparatus according to the invention; and
Fig. 2 is a block diagram showing the most important elements of an electronic controlling device for controlling the hemodialyzer apparatus according to the invention as shown in Fig. 1.

An embodiment of a hemodialyzer apparatus according to the invention shown in the figures of the drawing in strongly stylized shape comprises a number of elements, which are placed in connection with an apparatus front plate, namely a tube pump 9, which is placed in the blood channel system of the apparatus, which here in short is named the blood path of the apparatus, the tube pump is shown designed in a manner shown per se with one in a half circle internally along a half circle shaped wall traced tube, against which in radial direction are pressing along of two diametrically oppositely placed rolling devices, which by a drive motor is kept in rotation around the center of the half circle. The speed of the drive motor is stepless variable and is controlled as described in the following.

The inlet of the blood pump is connected to an arterial placed, e.g. cannula-, outlet BA, provided e.g. by catheter application and/or cannula insertion in the neck of a patient, collar-bone area or at another suitable connection area. The opposite end of the blood path of the apparatus is connected to a venous arranged, e.g. catheter- and/or cannula-, blood path inlet BB. The inlet BB can be arranged with a cannula placed elsewhere, but in practice special cannulas are used, to which a blood outtake pipe can be connected and a blood inlet pipe, as said cannula has two internal channels, where one debouches into the side area of the cannula in the blood vein of the patient, while the other debouches into the end of the cannula. In practice it is hereby possible hydraulically to achieve a safe and sufficient separation between the blood flow in the two pipes, out from and into the blood circulation of a patient.

Between the blood pump 9 and the connection end BB at 6 a connection for at pressure gauge 5 is arranged, while 7 indicates arrangement of an air detector known per se, which by presence of a certain maximum quantity of air in the blood path gives an e.g. electrical signal to one at 8 shown tube clamp known per se and at receiving the electrical signal the air detector 7 closes completely for the blood path by e.g. squeezing together the blood path channel at 8. Furthermore an artificial kidney or a filtration device 14 in the blood path channel are arranged.

The dialysis channel outlet or dialysate outlet of the artificial kidney or filtration device 14 is connected to a first dialysis pump 10, which can be of the same type as the blood pump 9, but is arranged having in the range of 1/10 maximum pump yield compared with the pump yield of the blood pump 9, allowing up to approximately 20 l blood to pass per hour retracted from a patient as described. Accordingly the dialysis pump 10 can suitably be arranged to let up to approximately 2 l dialysate pass per hour. As described in the preceding text the dialysis pump is driven, such that it is stepwise controllable in the yield area e.g. 0-2000 ml per hour. A step size of e.g. 1 ml is easily determined as a suitable value. The drive motor can e.g. be a step motor, the step speed of which is electronically controlled as further described in the following.

A similar second dialysis pump is arranged at 11. Said two dialysis pumps are each connected to a storage bag made of plastic sheet. The bags are suspended in a lever mechanism by a lever bar 12, which during operation of the apparatus by controlling of the second dialysis pump 11 in relation to a predetermined working speed for the first dialysis pump 10 and thereby controlling of the pump 11 in dependency of the changes in added dialysate quantity to the left bag is tried to be kept horizontal, such that an equal amount fresh dialysate preparation from the right bag is led to the artificial kidney or filtration device 14 and thereby in desired range via the artificial kidney or filtration device 14 to the blood circulation of the patient per time rate. It is possible to preset the apparatus such that the pump 10 has a desired higher yield than the pump 11. Thus a desired predetermined and controllable liquid draw from the patient is achieved resulting in an increase in weight of the added weights of DM + DL.

There is of course nothing preventing a drawing off of the bag contents of the left bag to a dialysate cleaning plant and further to the right bag during operation, as long as the two transport quantities per time unit are equal. But the idea of the apparatus is the availability of the possibility for the supply of ready made dialysate preparation via the pump 11 to the artificial kidney/filtration device 14.

It is noticed that the apparatus operates with separate passage channels in the apparatus for blood and dialysate. Passage between the two path systems only exists via the artificial kidney or via the transmission membrane of the filtration device.

However the apparatus is easy to change-over for other applications as described in the preceding. To achieve a lower pump yield when the patient is a child e.g. another pump insertion with a different and accordingly lower yield can easily be replaced in the pump housing from the front plate by opening of such a tube pumps inspection window.

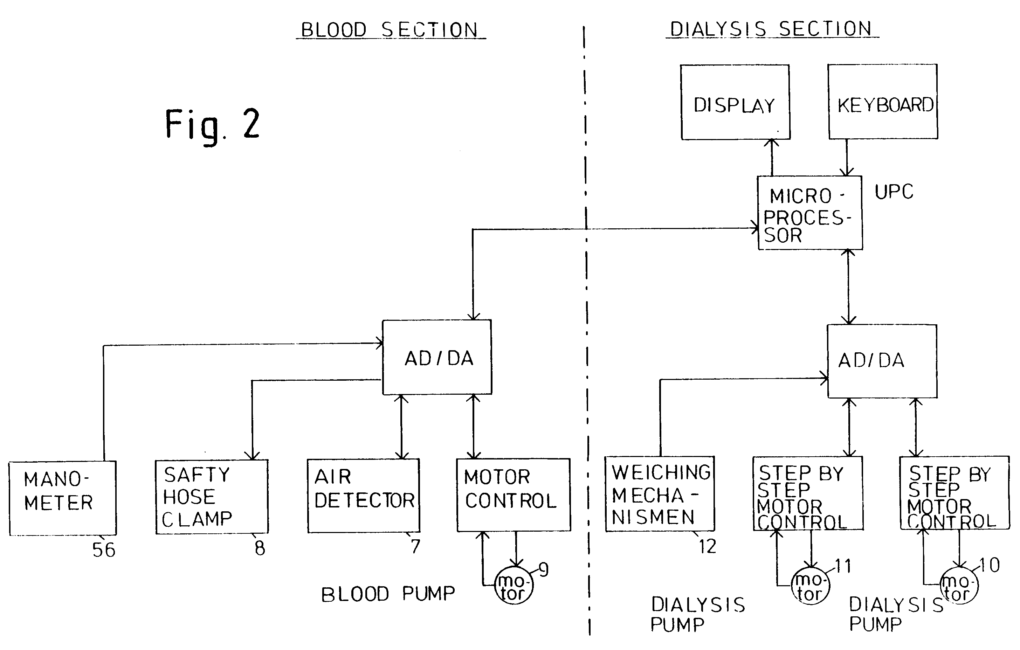

At the top of the front plate of the apparatus a main switch 1, an alarm indicator 3 as well as a display 2 respectively are arranged which in a text in a suitable language indicates the operation condition of the apparatus displayed e.g. by an electronic control system, which is shown schematically in Fig. 2 in the drawing. A key board 4 serves for operation of said control system.

As shown in Fig. 2 of the drawing the apparatus comprises a microprocessor UPC, controlling the two circulations of the apparatus, namely the dialysate path and the blood path. The microprocessor UPC is controlled in an electronically manner known per se by the key board 4 and providing indication via the display 2. Via an analog to digital converter, digital to analog converter respectively AD/DA the step motors are controlled, which powers the first 10 and the second dialysis pump 11. The weighing device 12, 13 generates state signals relating to the passing dialysate quantity per time unit by means of a simple weight determination respective by alarm at dialysate preparation deficiency. In practice the two pump motors will be controlled such that a suitable pressure difference is kept over the filtration members in the artificial kidney or filtration device 14.

Via similar art of analog to digital converter respectively digital to analog signal converter the blood pump 6 is controlled, likewise state signals are received from the air detector 7, the tube clamping device 8 and the pressure gauge 5,6. The microprocessor or the computer UPC controls the mutual relations between the electronic working components.

The display can from said electronic working components easily be brought to indicate: the flow quantity through the pumps, the quantity of the withdrawn liquid, deficiency of dialysate preparation, malfunction or choking of the pumps as well as an alarm call at opening of the protection cover over the pumps, etc., occurence of non-permissible air quantities in the blood path, exceeding of pressure limits at occurence of blood coagulation in the dialyse filter and/or tubes. In the latter case the blood pump could be stopped, and the error indicated. Furthermore inadequate (too low) volume passing the first dialysis pump in relation to the second dialysis pump can be detected, too large liquid draw and leaks in the dialysate circulation can also be detected.

Considering that a dialyzer apparatus must not break down, an applicable embodiment can be provided with not only one, but two microprocessors or computers operating independently, and where one monitors the controlling operations themselves, i.e. the first calculates and controls the pump speed and activates the tube clamping device 8, while the other monitors the pump speed, air detector, manometer and the weighing mechanisms, the other can also control the display. Alarm limits can be loaded and preadjusted by means of keyboard functions. At start of the apparatus the processors or the computer can be programmed to run a self controlling test as well as to carry out precontrol of the electronic components.

The tachometer mentioned, which can be arranged at the pumps, can e.g. be connected also to the other of the two microprocessors or computers.

## Claims

1. A hemodialyzer apparatus comprising an inlet (BA) for blood, an outlet (BB) for treated blood, a blood flow path with an adjustable blood pump (9), a pressure gauge (5,6) for monitoring the blood pressure, an air detector (7) for detecting air in-the blood flow path, an artificial kidney or filtration device (14), such as a filter column comprising at least one filter for passage of liquid, plasma or other substance from the blood, a dialysate channel connected to the dialysate outlet of the artificial kidney or filtration device, a receptacle (DM) for the dialysate and at least one adjustable dialysis pump, characterized in that the blood pump (9) is a stepless variable pump, and that the dialysis pump(s) (10;11) is a stepwise adjustable pump(s).

2. A hemodialyzer according to claim 1, characterized in that there are arranged two stepwise adjustable dialysis pumps (10,11) in the dialysis channel.

3. A hemodialyzer according to claim 2, characterized in that it comprises a weighing mechanism (12) connected with the receptacle (DM) receiving dialysate from the first dialysis pump (10) and for a storage receptacle (DL), for the fresh or cleansed dialysate, said receptacle being connected to the other dialysis pump (11).

4. A hemodialyzer according to claim 2, characterized in that the artificial kidney or filtration device (14) is provided with filter for plasmaforese and/or plasma replacement, and that the other dialysis pump (11) is adjusted synchronously with the first dialysis pump (10).

5. A hemodialyzer according to claim 2, characterized in that the artificial kidney or filtration device (14) has one for the purpose suitable, such as plasmaforese filtration providing, filter member, and an adsorption providing, such as immuno adsorption providing, such as antibodies and cholesterol removing, filter member supplied with dialysate from the first dialysis pump (10), the outlet of the filter member connected to the arterial inlet of the artificial kidney or filtration device (14).

6. A hemodialyzer according to claim 5, characterized in that the other dialysis pump (11) is arranged in the dialysate flow path between the dialysate outlet of the treated, possible further treated dialysate and the dialysate inlet of the artificial kidney or filtration device (14), and that the two dialysis pumps (10,11) are adjusted to work synchronously.

7. A hemodialyzer according to claims 1 or 2, characterized in that the artificial kidney or filtration device (14) is arranged to eliminate liquid, preferably water drawing, from the passing blood, and that the first dialysis pump (10) is connected to the receptacle (DM) connected with the weighing mechanism (12) for determination of received liquid volume.

8. A hemodialyzer according to claims 1,2,3,4,5,6 or 7, characterized in that in the blood flow path there is a shut off valve (8), such as a tube clamp, which controllingly is in connection with the air detector (7) and is closed by this (7), when presence of an air volume above a predetermined maximum is detected.

9. A hemodialyzer according to claims 1,2,3,4, 5,6,7, or 8 characterized in that weighing mechanism (12) for the receptacle (DL) with preparation is arranged with an alarm device (13), giving alarm for an outlet quantity, which is below a predetermined quantity, which should be conveyed to the dialysate flow path per time unit.

## Patentansprüche

1. Eine Hämodialysevorrichtung, umfassend einen Einlaß (BA) für Blut, einen Auslaß (BB) für behandeltes Blut, einen Blutflußweg mit einer einstellbaren Blutpumpe (9), ein Manometer (5,6) zum Beobachten des Blutdrucks, einen Luftdetektor (7) zum Feststellen von Luft im Blutflußweg, eine künstliche Niere oder eine Filtrationseinrichtung (14) wie eine Filtersäule, umfassend mindestens ein Filter zum Passieren von Flüssigkeit, Plasma oder anderer Substanz aus dem Blut, einen mit dem Dialysatauslaß der künstlichen Niere oder der Filtereinrichtung verbundenen Dialysatkanal, einen Behälter (DM) für das Dialysat und mindestens eine einstellbare Dialysepumpe (9), dadurch gekennzeichnet, daß die Blutpumpe (9) eine stufenlos variierbare Pumpe ist und daß die Dialysepumpe(n) (10;11) eine schrittweise einstellbare Pumpe ist/schrittweise einstellbare Pumpen sind.

2. Ein Hämodialysegerät nach Anspruch 1, dadurch gekennzeichnet, daß zwei schrittweise einstellbare Dialysepumpen (10,11) in dem Dialysekanal angeordnet sind.

3. Ein Hämodialysegerät nach Anspruch 2, dadurch gekennzeichnet, daß es eine Wägevorrichtung (12) umfaßt, verbunden mit dem Dialysat von der ersten Dialysepumpe (10) aufnehmenden Behälter (DM) und für einen Lagerbehälter (DL) für das frische oder gereinigte Dialysat, wobei der Behälter mit der anderen Dialysepumpe (11) verbunden ist.

4. Ein Hämodialysegerät nach Anspruch 2, dadurch gekennzeichnet, daß die künstliche Niere oder die Filtrationseinrichtung (14) mit einem Filter für Plasmaforese und/oder Plasmaersatz versehen ist, und daß die andere Dialysepumpe (11) synchron mit der ersten Dialysepumpe (10) eingestellt ist.

5. Ein Hämodialysegerät nach Anspruch 2, dadurch gekennzeichnet, daß die künstliche Niere oder die Filtrationseinrichtung (14) ein für den Zweck geeignetes - wie ein Plasmaforesefiltration zur Verfügung stellendes - Filterelement und ein Adsorption wie Immunoadsorption wie Antikörper- und Cholesterinentfernenung zur Verfügung stellendes Filterelement aufweist, das von der ersten Dialysepumpe (10) mit Dialysat versehen wird, wobei der Auslaß von dem Filterelement mit dem arterischen Einlaß der künstlichen Niere oder der Filtrationseinrichtung (14) verbunden ist.

6. Ein Hämodialysegerät nach Anspruch 5, dadurch gekennzeichnet, daß die andere Dialysepumpe (11) in dem Dialyseflußweg zwischen dem Dialysatauslaß des behandelten, möglichen weiter behandelten Dialysats und dem Dialysateinlaß der künstlichen Niere oder der Filtrationseinrichtung (14) angeordnet ist, und daß die zwei Dialysepumpen (10,11) so eingestellt sind, daß sie synchron arbeiten.

7. Ein Hämodialysegerät nach Anspruch 1 oder 2, dadurch gekennzeichnet, daß die künstliche Niere oder die Filtrationseinrichtung (14) so angeordnet ist, daß sie Flüssigkeit, vorzugsweise durch Wasserziehen, vom passierenden Blut eliminiert und daß die erste Dialysepumpe (10) mit dem Behälter (DM) verbunden ist, der mit der Wägevorrichtung (12) für die Bestimmung des erhaltenen Flüssigkeitsvolumens verbunden ist.

8. Ein Hämodialysegerät nach einem der Ansprüche 1, 2, 3, 4, 5, 6 oder 7, dadurch gekennzeichnet, daß in dem Blutflußweg ein Absperrventil (8) wie eine Schlauchklemme angeordnet ist, das/die mit dem Luftdetektor (7) in Steuer-Verbindung steht und durch diese (7) verschlossen wird, wenn die Gegenwart eines Luftvolumens über einem voreingestellten Maximalwert festgestellt wird.

9. Ein Hämodialysegerät nach einem der Ansprüche 1, 2, 3, 4, 5, 6, 7 oder 8, dadurch gekennzeichnet, daß die Wägevorrichtung (12) für den Behälter (DL) mit Präparat mit einer Alarmeinrichtung (13) angeordnet ist, die Alarm bei einer Ausflußmenge gibt, die unterhalb einer vorbestimmten Menge liegt, die dem Dialyseflußweg pro Zeiteinheit zugeführt werden soll.

## Revendications

1. Appareil d'hémodialyse comprenant une entrée (BA) pour le sang, une sortie (BB) pour le sang traité, une voie d'écoulement du sang avec une pompe de sang réglable (9), une jauge de pression (5,6) pour contrôler la pression sanguine, un détecteur d'air (7) pour détecter de l'air dans la voie d'écoulement du sang, un dispositif de filtration ou rein artificiel (14), tel qu'une colonne de filtration comprenant au moins un filtre pour le passage de liquide, plasma ou autre substance provenant du sang, un canal de dialysat relié à la sortie de dialysat du dispositif de filtration ou du rein artificiel, un réceptacle (DM) pour le dialysat et au moins une pompe de dialyse réglable, caractérisé en ce que la pompe de sang (9) est une pompe variable sans discontinuité, et en ce que la ou les pompes de dialyse (10,11) est/sont une ou des pompes réglables pas à pas.

2. Appareil d'hémodialyse selon la revendication 1,
caractérisé en ce qu'il est agencé deux pompes de dialyse réglables pas à pas (10,11) dans le canal de dialyse.

3. Appareil d'hémodialyse selon la revendication 2,
caractérisé en ce qu'il comprend un mécanisme de pesage (12) relié au réceptacle (DM) recevant le dialysat de la première pompe de dialyse (10) et pour un réceptacle de stockage (DL), pour le dialysat frais ou purifié, ledit réceptacle étant relié à l'autre pompe de dialyse (11).

4. Appareil d'hémodialyse selon la revendication 2,
caractérisé en ce que le dispositif de filtration ou le rein artificiel (14) est muni d'un filtre pour la plasmaphérèse et/ou le remplacement du plasma, et en ce que l'autre pompe de dialyse (11) est réglée en synchronisme avec la première pompe de dialyse (10).

5. Appareil d'hémodialyse selon la revendication 2,
caractérisé en ce que le dispositif de filtration ou le rein artificiel (14) présente un organe de filtration approprié dans le but tel que fournir la filtration de plasmaphérèse et un organe de filtration fournissant l'adsorption, tel que fournissant une immuno-adsorption, telle que l'élimination d'anticorps et du cholestérol, alimenté en dialysat à partir de la première pompe de dialyse (10), la sortie de l'organe de filtration étant reliée à l'entrée artérielle du dispositif de filtration ou du rein artificiel (14).

6. Appareil d'hémodialyse selon la revendication 5,
caractérisé en ce que l'autre pompe de dialyse (11) est agencée dans la voie d'écoulement du dialysat entre la sortie de dialysat du dialysat traité, éventuellement encore traité, et l'entrée de dialysat du dispositif de filtration ou du rein artificiel (14), et en ce que les deux pompes de dialyse (10,11) sont réglées pour travailler de façon synchrone.

7. Appareil d'hémodialyse selon les revendications 1 ou 2,
caractérisé en ce que le dispositif de filtration ou le rein artificiel (14) est agencé pour éliminer du liquide, de préférence aspirer de l'eau, du sang qui circule, et en ce que la première pompe de dialyse (10) est reliée au réceptacle (DM) relié au mécanisme de pesage (12) pour la détermination du volume de liquide reçu.

8. Appareil d'hémodialyse selon les revendications 1, 2, 3, 4, 5, 6 ou 7,
caractérisé en ce que, dans la voie d'écoulement du sang, il est prévu une vanne d'arrêt (8), telle qu'une pince de tube, qui est en liaison de commande avec le détecteur d'air (7) et est fermée par celui-ci (7) quand la présence d'un volume d'air au-dessus d'un maximum prédéterminé est détectée.

9. Appareil d'hémodialyse selon les revendications 1, 2, 3, 4, 5, 6, 7 ou 8,
caractérisé en ce que le mécanisme de pesage (12) pour le réceptacle (DL) avec une préparation est muni d'un dispositif d'alarme (13), fournissant une alarme pour une quantité de sortie, qui est au-dessous d'une quantité prédéterminée, qui doit être transportée vers la voie d'écoulement de dialysat par unité de temps.
